# EUROPÄISCHE PATENTANMELDUNG

(11) **EP 3 811 853 A1**
(43) Veröffentlichungstag der Anmeldung: **28.04.2021**
(21) Anmeldenummer: 19204274.5
(22) Anmeldetag: 21.10.2019
(51) Int. Cl.: A61B 5/00, A61M 1/12

(54) **ART UND WEISE DER ALARMIERUNG EINES HERZUNTERSTÜTZUNGSSYSTEMS**

(71) Anmelder: Berlin Heart GmbH, 12247 Berlin (DE)
(72) Erfinder: FRISCHKE, Dr.-Ing. Michael, 15834 Rangsdorf (DE); JANKOWSKY, Florian, 15848 Friedland (DE)
(74) Vertreter: Pfenning, Meinig & Partner mbB

(57) **Zusammenfassung**

Herzunterstützungssystem (1) mit einer Steuereinheit (2) und Schallerzeugungsvorrichtungen (4), wobei die Steuereinheit (2) dazu ausgebildet ist, Alarmereignisse (43) zu erkennen und zu verarbeiten und Steuerungsaufgaben im Herzunterstützungssystem (1) auszuführen, gekennzeichnet durch eine Speichervorrichtung (6), die dazu ausgebildet ist, ein oder mehrere vordefinierte Alarmsignalzeitverläufe (45) und eine oder mehrere Alarmprioriten (42) so zu speichern, dass jeweils ein Alarmsignalzeitverlauf (45) und jeweils eine Alarmpriorität (42) einem Alarmereignis (43) zugeordnet sind, eine Schallerzeugungssteuervorrichtung (8), die dazu ausgebildet ist, bei Erkennung eines Alarmereignisses (43) dem dem Alarmereignis (43) in der Speichervorrichtung (6) zugeordneten Alarmsignalzeitverlauf (45) einen Lautstärkezeitverlauf aufzuprägen, der von der in der Speichervorrichtung (6) dem jeweiligen Alarmereignis (43) zugeordneten Alarmpriorität (42) und der Zeitdauer ab Erkennung des Alarmereignisses (43) abhängig ist, die vorgenannte Schallerzeugungssteuervorrichtung (8), die dazu ausgebildet ist, bei Erkennung eines Alarmereignisses (43) die Schallerzeugungsvorrichtungen (4) so anzusteuern, dass sie den vorgenannten Alarmsignalzeitverlauf (45) mit dem aufgeprägten Lautstärkezeitverlauf in Schallsignale umsetzen.

## Beschreibung

Die Schutzrechtsanmeldung betrifft eine Alarmierungsvorrichtung in Herzunterstützungssystemen, insbesondere in Herzunterstützungssystemen mit einem an den Kreislauf angeschlossenen Pumpensystem zur Aufrechterhaltung des Blutkreislaufes. Die Ausgabe von Informationen über den Zustand des Patienten aber auch des Gerätes an das medizinische Personal oder medizinische Überwachungssysteme ist in Herzunterstützungssystemen von hoher Bedeutung. Insbesondere wichtig sind hierbei akustische Signale, da sie ohne direkten Blickkontakt und auch aus größerer Distanz wahrgenommen werden können.

Eine mögliche akustische Alarmierungsvorrichtung für Herzpumpen wird in der Patentschrift EP1812094B1 vorgestellt. Das hierin beschriebene Pumpensteuersystem umfasst unter anderem eine Vorrichtung für das Empfangen und Ausgeben von Audiosignalen.

Die Aufgabe des beanspruchten Herzunterstützungssystems besteht darin, die bestehenden Lösungen für Herzunterstützungssysteme zu verbessern. Diese Aufgabe wird mit einer Anordnung nach einem der Ansprüche 1 bis 13 und dem Verfahren nach Anspruch 14 gelöst. Insbesondere wird die Aufgabe dadurch gelöst, dass dem Zeitverlauf des jeweiligen Alarmsignals ein Lautstärkezeitverlauf aufgeprägt wird, der, neben der Zeitdauer seit Eintreffen eines Alarmereignisses, auch von der dem Alarmereignis zugeordneten Alarmpriorität abhängt.

Unter dem Begriff Ereignis wird das Eintreten eines bestimmten Zustandes verstanden. Das Eintreten eines Zustandes wird im Allgemeinen mit Hilfe eines Vergleichs gegen einen Referenzwert festgestellt. Das Vergleichsergebnis beschreibt den festgestellten Zustand und somit das Ereignis. Referenzwerte können, abhängig vom Kontext, zeitlich fest oder aber auch zeitlich variabel sein. Mit der Feststellung des Vergleichsergebnisses ist das Ereignis im Herzunterstützungssystem eingetroffen beziehungsweise wurde das Ereignis vom Herzunterstützungssystem erkannt. Häufig erhalten Ereignisse vordefinierte Bezeichner, beispielsweise charakterisierende Klarnamen oder auch Nummern, um sie innerhalb des Herzunterstützungssystems, beispielsweise mit einem Softwareprogramm, verarbeiten zu können. Eine Verarbeitung durch eine elektronische Schaltung ist jedoch prinzipiell auch möglich.

Zustände im Herzunterstützungssystem werden beispielsweise definiert als vorzeichenbehaftete Werte von elektrischen Spannungen oder elektrischen Strömen, die zwischen oder an vordefinierten Punkten gemessen werden können. Der Vergleich der gemessenen Spannungen und Ströme mit Referenzwerten führt dazu, dass das Eintreten eines jeweiligen Zustandes festgestellt werden kann. Die elektrischen Spannungen oder elektrischen Ströme können beispielsweise von Sensoren generiert werden, wobei die generierten Spannungen oder Ströme zu physikalische Größen wie Druck, Dehnung, Durchfluss, Länge, Licht, Schall, Beschleunigung, Magnetfeld oder chemische Zusammensetzungen von Stoffen korrespondieren können. Elektrische Spannungen oder Ströme können aber auch direkt von biologischen Vorgängen generiert und mit Hilfe von Elektroden abgeleitet und dem Herzunterstützungssystem an elektrischen Eingängen zugeführt werden. Beispiele hierfür sind Elektrokardiogrammsignale, das Elektroenzephalogrammsignale oder auch das Elektromygrammsignale. Häufig werden die Amplituden der Sensorspannungen oder -ströme zunächst mit einem Verstärker erhöht, bevor sie am Herzunterstützungssystem anliegen und verarbeitet werden.

Neben sensorischen und biologischen Ereignisquellen kann das Herzunterstützungssystem selbst Quelle von Ereignissen sein und Ereignisse durch interne elektronische Komponenten oder die Steuereinheit des Herzunterstützungssystems erzeugen, beispielsweise mit elektrischen Spannungen, die am Ausgang eines Zeitgebers, eines Zählers, einer Uhr oder eines Oszillators entstehen oder auch Spannungen, die von Komponenten des Herzunterstützungssystems erzeugt werden, um einen jeweiligen internen Zustand anzuzeigen. Darüber hinaus können auch Softwarekomponenten Ereignisse erzeugen, in dem beispielsweise Berechnungsergebnisse oder Ergebnisse logischer Verknüpfungen als Zustand betrachtet werden, dessen Eintreffen durch den Vergleich mit einem Referenzwert festgestellt wird.

Ereignisse sind im Herzunterstützungssystem im Allgemeinen vordefiniert, so dass auf ein vordefiniertes Ereignis eine vordefinierte Reaktion des Herzunterstützungssystems erfolgen kann. Alarmereignisse sind spezielle Ereignisse, bei denen als Reaktion auf ihr Eintreffen im Herzunterstützungssystem unter anderem ein vordefinierter Alarm ausgelöst werden soll. Alarmereignisse können den Patienten und seinen Zustand betreffen, aber auch technische Ereignisse wie beispielsweise den Ausfall einer Steuereinheit oder einer elektrischen Spannungsquelle anbelangen.

Beispiele für Ereignisse, die Alarme auslösen, sind unter anderem der Ausfall einer kritischen Komponente des Herzunterstützungssystems, ein Abfall des Blutflusses, die Entfernung mehrerer Spannungsquellen oder auch die signifikante Abweichung kritischer Sensorwerte von ihren Normwerten. Beispiele für Ereignisse, die keine Alarme auslösen, sind unter anderem die vollzogene Aufladung eines internen Akkumulators, die Bedienung des Displays oder eines Tasters durch einen Nutzer, Änderungen von Sensorwerten innerhalb von Toleranz- oder Alarmgrenzen - beispielsweise die Änderung eines Motorstroms im kleinen Rahmen. Weitere Beispiele für Ereignisse ohne Alarmauslösung beinhalten unter anderem das Eintreten vordefinierter unkritischer physiologischer Zustände, die lediglich in einem Logbuch gespeichert werden und später von einem Arzt ausgelesen und geprüft werden sollen, sowie Änderungen des Betriebmodus des Herzunterstützungssystems.

Das beanspruchte Herzunterstützungssystem enthält eine erste Steuereinheit und mindestens eine erste Schallerzeugungsvorrichtung. Die Steuereinheit ist dazu ausgebildet, Ereignisse und insbesondere Alarmereignisse zu erkennen und zu verarbeiten sowie Daten zu verarbeiten und Steuerungsaufgaben im Herzunterstützungssystem auszuführen. Bei einer Steuereinheit kann es sich um einen Mikrocontroller, einen Mikroprozessor aber auch um eine elektronische Schaltung handeln, die mehrere Komponenten enthält und dazu ausgebildet ist, Steuerungsaufgaben zu übernehmen.

Das Herzunterstützungssystem enthält außerdem eine Speichervorrichtung und eine Schallerzeugungssteuerung. Bei der Speichervorrichtung kann es sich um RAM-Baugruppen (RAM: Random Access Memory) handeln oder aber auch um ROM-Baugruppen (ROM: Read Only Memory). Darüber hinaus sind andere Speicherformen wie EPROM (Erasable Programmable Read Only Memory), EEPROM (Electrically Erasable Programmable Read Only Memory), FPGA (Field Programmable Gate Array) oder aber auch magnetische oder Solid-State Festplatten als Speicher denkbar. Die Speichervorrichtung ist dazu ausgebildet, ein oder mehrere vordefinierte Alarmsignalzeitverläufe und eine oder mehrere Alarmprioritäten so zu speichern, dass jeweils ein Alarmsignalzeitverlauf und jeweils eine Alarmpriorität einem oder mehreren vordefinierten Alarmereignissen zugeordnet sind. Die Zuordnung kann beispielsweise über eine im Speicher abgelegte Tabelle realisiert werden, in der in jeder Zeile ein Alarmereignis sowie die zugeordnete Alarmpriorität sowie ein Zeiger auf einen Speicherbereich eingetragen ist, der den dem Alarmereignis zugeordneten Alarmsignalzeitverlauf enthält. Alternativ dazu kann die Zuordnung auch über eine im Speicher abgelegte Datenbank realisiert werden. Diese beiden Realisierungsmöglichkeiten stellen jeweils nur Beispiele dar und keineswegs eine vollständige Aufzählung möglicher Realisierungsformen.

Ein Alarmsignalzeitverlauf kann beispielsweise als eine Abfolge von gewichteten Frequenzen oder gewichteten Frequenzkombinationen gespeichert werden. Dies kann beispielsweise mit einer Abfolge von Zusammenstellungen geschehen, wobei jede Zusammenstellung eine Zeitdauer, eine Frequenzliste und eine Liste mit den Lautstärken der in der Frequenzliste enthaltenen Frequenzen enthält. Alternativ ist beispielsweise die Speicherung des Alarmsignalzeitverlaufs in Form eines abgetasteten Zeitverlaufs möglich. Weitere Möglichkeiten, den Zeitverlauf von Alarmsignalen zu speichern, sind prinzipiell denkbar, so dass die angegebenen Möglichkeiten lediglich als Beispiel zu verstehen sind.

Eine Alarmpriorität gibt die Wichtigkeit eines Alarmereignisses wieder. Diese Wichtigkeit wird mit einem Element einer endlichen Menge angegeben, wobei diese endliche Menge häufig nur wenige vordefinierte Elemente umfasst. Beispiele für solche eine solche Mengen sind M = {hohe Priorität; mittlere Priorität; geringe Priorität} oder auch M = {1; 2; 3; 4}.

Beispiele für wichtige Alarmereignisse in Herzunterstützungssystemen, d.h. Alarmereignisse hoher Priorität, sind unter anderem der Ausfall einer kritischen Komponente des Herzunterstützungssystems, ein Abfall des Blutflusses, die Entfernung mehrerer Spannungsquellen oder auch die signifikante Abweichung kritischer Sensorwerte von ihren Normwerten. Beispiele für weniger wichtige Alarmereignisse in Herzunterstützungssystemen, d.h. Alarmereignisse mittlerer oder geringer Priorität, sind beispielsweise ein niedriger Ladestatus einer Batterie, das Abstecken einer Spannungsquelle, der Ausfall einer unkritischen Komponente des Herzunterstützungssystems oder aber auch Abweichungen unkritischer Sensorwerte von ihren Normwerten.

Die Schallerzeugungssteuerung kann Teil der Steuerung des Herzunterstützungssystems sein. Beispielsweise kann sie als Prozess oder Funktionsmodul in der Software der Steuerung implementiert sein und auch Zugriff auf die elektrischen Anschlüsse der Steuerung besitzen. Alternativ dazu besteht auch die Möglichkeit, die Schallerzeugungssteuerung als Hardwaremodul innerhalb der Steuerung des Herzunterstützungssystems, als eigenständige elektronische Schaltung oder mit einem separaten Mikrocontroller zu realisieren. Die Schallerzeugungssteuerung erhält beispielsweise von der Steuerung des Herzunterstützungssystems die Information über das Eintreffen eines Alarmereignisses und um welches Alarmereignis es sich handelt.

Die Schallerzeugungssteuerung des Herzunterstützungssystems ist dazu ausgebildet, bei Eintreffen eines Alarmereignisses dem dem Alarmereignis in der Speichervorrichtung zugeordneten Alarmsignalzeitverlauf einen jeweils vordefinierten Lautstärkezeitverlauf aufzuprägen, der von der in der Speichervorrichtung dem jeweiligen Alarmereignis zugeordneten Alarmpriorität und der Zeitdauer ab Eintreffen des Alarmereignisses abhängig ist. Dazu ruft die Schallerzeugungssteuerung die Alarmpriorität des jeweilig eingetroffenen Ereignisses und den Alarmsignalzeitverlauf aus der Speichervorrichtung ab, um anschließend den Lautstärkezeitverlauf des Alarmsignals auf vordefinierte Weise zu modifizieren. Die Modifikation erfolgt beispielsweise so, dass die Lautstärke des Alarmsignalzeitverlaufs in einem vordefinierten, von der Alarmpriorität abhängigen, An-Aus-Muster verändert wird. An-Aus-Muster bedeutet, dass die Lautstärke des Signals auf vordefinierte Art und Weise mehrfach vom im Speicher abgelegten Wert auf Null wechselt und umgekehrt. Der Zeitabschnitt, bei dem der Lautstärkewert bei Null liegt, wird als Aus-Phase bezeichnet, die anderen Zeitabschnitte als An-Phase. Die Lautstärke des Alarmsignals wird während der An-Phasen darüber hinaus mit zunehmender Zeit bis hin zu einem Maximalwert gesteigert.

Die Schallerzeugungssteuerung ist außerdem dazu ausgebildet, bei Eintreffen eines Alarmereignisses eine oder mehrere Schallerzeugungsvorrichtungen so anzusteuern, dass sie die vorgenannten Alarmsignalzeitverläufe mit dem vorgenannten aufgeprägten Lautstärkezeitverlauf in Schallsignale umsetzen.

Die Schallerzeugungsvorrichtung kann dazu beispielsweise eine Digital-Analog-Umsetzer-Einheit, einen Verstärker sowie einen Schallwandler, beispielsweise einen Breitbandlautsprecher, wie sie aus der Hifi-Technik oder Telefonanwendungen zur Sprachschallerzeugung bekannt sind, oder auch einen schmalbandigen Lautsprecher, wie beispielsweise einen Piezo-Buzzer, enthalten. Breitbandige Lautsprecher sind durch einen Frequenzgang gekennzeichnet, der in großen Teilen des menschlichen Hörbereichs Schallsignale abgeben kann. Schmalbandige Lautsprecher hingegen sind gekennzeichnet durch eine charakteristische Frequenz, mit der sie Schall abstrahlen können; andere Schallfrequenzen können hingegen nur mit geringer Leistung abgestrahlt werden. Sollen unter Nutzung von schmalbandigen Lautsprechern mehrere verschiedene Schallfrequenzen abgestrahlt werden, so können mehrere schmalbandige Lautsprecher mit verschiedenen charakteristischen Frequenzen kombiniert werden, wobei die Ansteuerung für jede Frequenz bzw. jeden schmalbandigen Lautsprecher separat von anderen Frequenzen bzw. schmalbandigen Lautsprechern erfolgen kann.

Die Ansteuerung des Digital-Analog-Umsetzers durch die Schallerzeugungssteuerung erfolgt so, dass die Schallerzeugungssteuerung das abgetastete und in seiner Lautstärke modifizierte Alarmsignal als Eingangssignal an den Digital-Analog-Umsetzer gesendet wird und der Digital-Analog-Umsetzer so angesteuert wird, dass er das empfangene Signal in ein zum Eingangssignal korrespondierendes Spannungssignal umsetzt und welches anschließend verstärkt an den nachgeschalteten Schallwandler weitergeleitet wird.

Alternativ kann die Schallerzeugungsvorrichtung auch realisiert werden über eine oder eine Kombination mehrerer elektronischer Oszillatorschaltungen, wobei jeder Oszillator ein Spannungssignal mit einer feststehenden Frequenz erzeugt. Die Schallerzeugungssteuerung steuert die Oszillatoren so an, dass die im Alarmsignalzeitverlauf festgelegten Frequenzen generiert werden und in einer weiteren Stufe entsprechend der im Alarmsignalzeitverlauf festgelegten, und entsprechend der Alarmpriorität und Zeitdauer modifizierten, Lautstärkewerten additiv kombiniert werden. Zur Erreichung eines angestrebten Lautstärkepegels kann das erzeugte Alarmsignal optional weiter verstärkt werden, bevor es an einen oder mehrere Schallwandler übergeben wird.

Das Herzunterstützungssystem umfasst optional eine Herzpumpe und eine Steuereinheit, die das Herzunterstützungssystem einschließlich der Herzpumpe steuert.

Ein weiteres, optionales Merkmal des beanspruchten Herzunterstützungssystems, welches die Ausfallsicherheit des Gesamtsystems erhöht, ist mindestens eine zweite, von der ersten Schallerzeugungsvorrichtung getrennt ansteuerbare, elektrisch betriebene Schallerzeugungsvorrichtung. Alle Schallerzeugungsvorrichtungen zusammen können mindestens einen Breitbandlautsprecher und mindestens einen schmalbandigen Lautsprecher enthalten. Optional sind jedoch auch ausschließlich breitbandige Lautsprecher oder auch ausschließlich schmalbandige Lautsprecher möglich.

Das beanspruchte Herzunterstützungssystem kann optional mindestens eine zweite Schallerzeugungssteuerung enthalten, die dazu ausgebildet ist, einen Funktionsausfall der ersten Schallerzeugungssteuerung zu detektieren und mindestens eine Schallerzeugungsvorrichtung so anzusteuern, dass die Schallerzeugungsvorrichtung vordefinierte Schallsignale erzeugt. Eine Möglichkeit, den Funktionsausfall der ersten Schallerzeugungssteuerung zu detektieren besteht darin, dass die erste Schallerzeugungssteuerung im Normalbetrieb an einem elektrischen Ausgang ein mit einem vordefinierten Muster gepulstes Spannungssignal erzeugt, dass von der zweiten Schallerzeugungssteuerung an einem elektrischen Eingang ausgewertet wird. Diese Auswertung kann beispielsweise von einem einfachen Mikrocontroller oder aber auch einer elektronischen Schaltung ausgeführt werden. Die zweite Schallerzeugungssteuerung kann so ausgebildet werden, dass sie eine Abweichung vom vordefinierten Muster, beispielsweise das Ausbleiben eines Pulses, erkennt und daraufhin ein Steuersignal ausgibt, dass die elektrische Ansteuerung eines Lautsprechers freischaltet. Die vorgenannte elektrische Ansteuerung eines Lautsprechers kann durch eine elektronische Oszillatorschaltung realisiert werden, mit der ein gepulstes elektrisches Spannungssignal erzeugt wird, das, sobald es an einen Lautsprecher durchgeleitet wird, zur Erzeugung eines dazu korrespondierenden Schallsignals führt.

Das Herzunterstützungssystem kann optional eine zweite elektrische Spannungsquelle enthalten, die dazu ausgebildet ist, bei Ausfall der ersten elektrischen Spannungsquelle mindestens eine Schallerzeugungsvorrichtung und die zweite Schallerzeugungssteuerung mit elektrischer Spannung zu versorgen. Diese Sicherheitsvorrichtung ist dafür bestimmt, den Ausfall der Hauptspannungsversorgung zu erkennen und gleichzeitig aber dafür zu sorgen, dass das Herzunterstützungssystem diesen Fehlerzustand akustisch melden kann. Beispielhaft ist diese optionale Funktionalität mit einer einfachen Diodenschaltung oder aber auch mit integrierten Schaltungen möglich.

Ein optionales Element des beanspruchten Herzunterstützungssystems ist eine Schallabstrahlungsvorrichtung, die dazu ausgebildet ist, die Schallabstrahlung zu beeinflussen. Hierbei kann es sich beispielsweise um ein Lochfeld oder eine Anordnung handeln, die aus mehreren parallel angeordneten schlitzförmigen Öffnungen besteht. Dabei sind bei Lochfeldern die Öffnungsdurchmesser nicht kleiner 2mm und bei Schlitzstrukturen die Schlitzbreiten nicht kleiner als 1.5mm. Darüber hinaus ist der Winkel γ zwischen der Hauptrichtung der Schallabstrahlung durch die Schallabstrahlungsvorrichtung und dem vom Display wegzeigenden Normalenvektor des Displays optional kleiner oder gleich 90°. Da das Display im Allgemeinen vom Träger des Herzunterstützungssystems weg zeigt, erfolgt die Schallabstrahlung somit im Wesentlichen auch vom Träger weg.

Ferner kann das Schallsignal optional durch eine Schallbeeinflussungsvorrichtung beeinflusst werden, die weitere Elemente enthält, beispielsweise Resonanzräume oder aber auch Abdeckungen, Folien oder auch Vlies im Schallausbreitungspfad. Auf diese Weise erfolgt eine Beeinflussung der Gewichtung der Schallfrequenzen.

Darüber hinaus enthält das beanspruchte Herzunterstützungssystem optional eine Drahtlosschnittstelle, die dazu ausgebildet ist, mit der Drahtlosschnittstelle einer externen Einheit Daten auszutauschen. Die externe Einheit weist optional eine optische Signalisierungseinheit auf. Optional kann die externe Einheit auch eine akustische Signalisierungseinheit enthalten. Bei den Drahtlosschnittstelle kann es sich beispielsweise um WLAN-Schnittstellen, Blutooth-Schnittstellen, Nahfunkschnittstellen oder aber auch um eine zelluläre Funkschnittstellen handeln, zum Beispiel für die Standards GSM, GPRS, EDGE, CDMA, UMTS, HSDPA, HSPA, LTE oder 5G.

Das beanspruchte Verfahren zu Alarmierung besitzt als ersten Schritt das Erkennen eines Alarmereignisses durch das Herzunterstützungssystem. Die Erkennung kann sowohl durch ein Software-Programm als auch durch eine elektronische Schaltung realisiert werden. Dabei erfolgt beispielsweise ein Vergleich eines an den Systemeingängen anliegenden Spannungs- oder Stromwertes mit einem Referenzwert oder alternativ ein Vergleich eines berechneten oder durch logische Verknüpfung ermittelten Wertes mit einem Referenzwert. Die Art des Alarmereignisses wird anhand der Art des durchgeführten Vergleichs festgelegt.

In einem zweiten Verfahrensschritt wird aus einer Speichervorrichtung die dem Alarmereignis zugeordnete Alarmpriorität und der dem Alarmereignis zugeordnete Alarmsignalzeitverlauf abgerufen.

In einem dritten Verfahrensschritt wird dem abgerufenen Alarmsignalzeitverlauf, in Abhängigkeit von der Alarmpriorität und von der Zeitdauer seit Eintreffen des Alarmereignisses, ein vordefinierter Lautstärkezeitverlauf aufgeprägt. Diese Modifikation des Alarmzeitverlaufs erfolgt beispielsweise so, dass die Lautstärke des Alarmsignalzeitverlaufs in einem vordefinierten, von der Alarmpriorität abhängigen, An-Aus-Muster verändert wird. An-Aus-Muster bedeutet, dass die Lautstärke des Signals auf vordefinierte Art und Weise mehrfach vom im Speicher abgelegten Wert auf Null wechselt und umgekehrt. Die Zeitabschnitte, in denen der Lautstärkewert bei Null liegt, werden jeweils als Aus-Phase bezeichnet, die anderen Zeitabschnitte jeweils als An-Phase. Außerdem wird die Lautstärke des Alarmsignals so modifiziert, dass die Lautstärke während der An-Phasen mit zunehmender Zeit gemessen ab Erkennen des Alarmereignisses bis hin zu einem Maximalwert ansteigt.

In einem vierten Verfahrensschritt erfolgt die Ansteuerung der Schallerzeugungsvorrichtung so, dass der Alarmsignalzeitverlauf mit dem aufgeprägten Lautstärkezeitverlauf in ein Schallsignal umgesetzt wird.

Im Folgenden werden anhand von Figuren Ausführungsbeispiele gezeigt und nachfolgend erläutert. Dabei zeigt
Figur 1: einen Systemüberblick über die grundlegenden alarmrelevanten Komponenten des beanspruchten Herzunterstützungssystems,
Figur 2: eine schematische Darstellung der Speichervorrichtung mit der Zuordnung vordefinierter Alarmprioritäten und vordefinierter Alarmsignalzeitverläufe zu vordefinierten Alarmereignissen,
Figur 3: einen Systemüberblick über die grundlegenden alarmrelevanten Komponenten des beanspruchten Herzunterstützungssystems einschließlich optionaler Komponenten,
Figur 4: eine schematische Darstellung der Schallabstrahlung,
Figur 5: eine schematische Realisierungsvariante für die Zusammenschaltung einer ersten und einer zweiten Spannungsquelle,
Figur 6: Realisierungsmöglichkeiten für Schallabstrahlungsvorrichtungen,
Figur 7: die Verfahrensschritte zum Abrufen und zur Modifikation der Alarmsignalzeitverläufe.

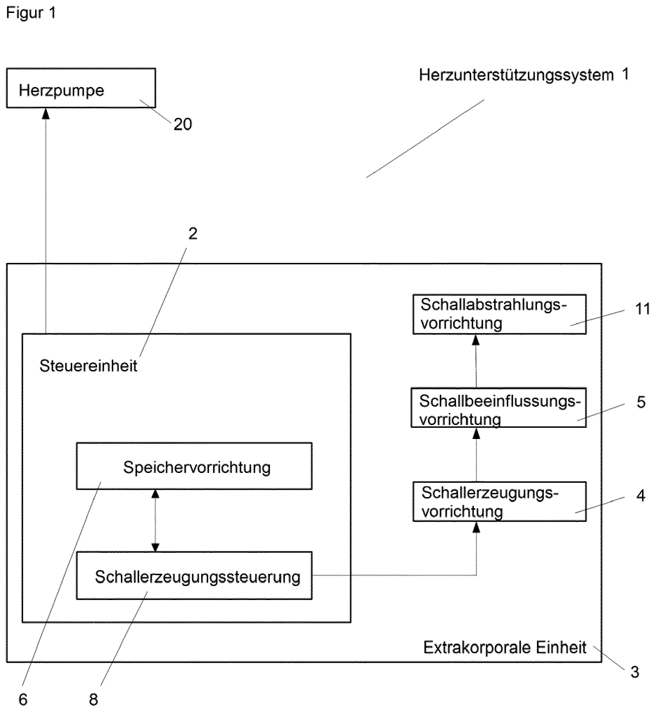

In Figur 1 werden die grundlegenden alarmrelevanten Komponenten des Herzunterstützungssystems 1 im Systemüberblick dargestellt. Dabei ist die Herzpumpe 20 beispielsweise im Körper eines Patienten angeordnet, während die anderen dargestellten Elemente in einer extrakorporalen Einheit 3 zusammengefasst werden. Beispielsweise können sich alle Elemente der extrakorporalen Einheit außerhalb des Körpers des Patienten in einem Gehäuse befinden. Zentrales Element des Herzunterstützungssystems 1 ist die Steuereinheit 2. Die Steuereinheit 2 ist dazu ausgebildet, Ereignisse und insbesondere Alarmereignisse 43 zu erkennen und zu verarbeiten sowie Daten zu verarbeiten und Steuerungsaufgaben im Herzunterstützungssystem auszuführen und zu koordinieren. Zu ihren Steuerungsaufgaben zählt unter anderem die Steuerung der Herzpumpe 20. Bei einer Steuereinheit 2 kann es sich um einen Mikrocontroller, einen Mikroprozessor aber auch um eine elektronische Schaltung handeln, die mehrere Komponenten enthält und dazu ausgebildet ist, Steuerungsaufgaben zu übernehmen. Die Schallerzeugungssteuerung 8 kann als Teil der Steuereinheit realisiert werden, beispielsweise auf einem eigenen Prozessorkern oder aber auch als Softwareprozess oder Funktionsmodul innerhalb der Software. Prinzipiell ist jedoch auch eine separate Realisierung in einem dafür ausgebildeten Mikrocontroller möglich.

Mit der Schallerzeugungssteuerung 8 ist eine elektronische Speichervorrichtung 6 verbunden, die Alarmsignalzeitverläufe und Alarmprioritäten sowie deren Zuordnung zu Alarmereignissen speichert.

Die Schallerzeugungsvorrichtung 4 wird durch die Schallerzeugungssteuerung 8 gesteuert. Sie umfasst beispielsweise einen Digital-Analog-Umsetzer, einen Verstärker und einen Lautsprecher. Der von der Schallerzeugungsvorrichtung erzeugte Schall wird in seiner Frequenzzusammensetzung von der Schallbeeinflussungsvorrichtung 4 beeinflusst. Zur Schallbeeinflussungsvorrichtung 4 zählen Abdeckungen, Folien oder aber auch Resonanzräume, die einen Einfluss auf das Schallsignal ausüben. Folien und Abdeckungen können neben der Schallbeeinflussungsfunktion auch eine Schutzfunktion ausüben, beispielsweise um das Herzunterstützungssystem vor Feuchtigkeit oder Partikeln zu schützen. Ist die spezifische Art des Einflusses der Schallbeeinflussungsfunktion bekannt, kann er durch die Schallerzeugungsvorrichtung 4 im Zuge der Schallsignalerzeugung berücksichtigt werden, beispielsweise durch ergänzende digitale oder analoge Filterung oder durch Adaption des in der Speichervorrichtung abgelegten Alarmzeitverlaufs.

Die Schallabstrahlungsvorrichtung 11 umfasst Bauelemente, die die Schallabstrahlung beeinflussen, beispielsweise Horngeometrien, Lochfelder oder Schlitzstrukturen. Dabei sind bei Lochfeldern die Öffnungsdurchmesser nicht kleiner 2mm und bei Schlitzstrukturen die Schlitzbreiten nicht kleiner als 1.5mm zu wählen.

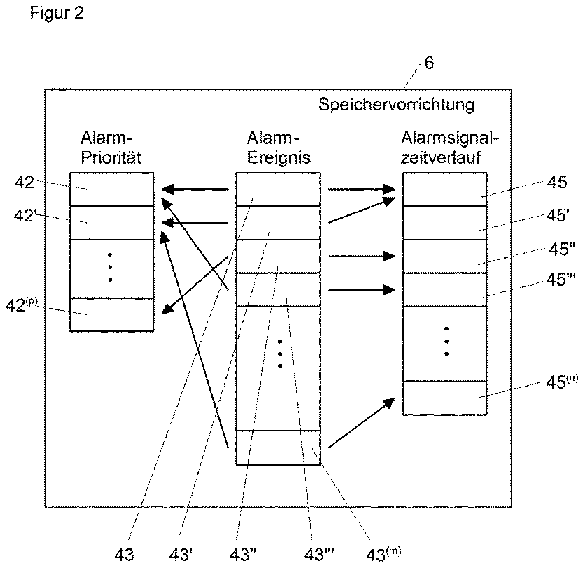

Figur 2 zeigt die Speichervorrichtung 6. Sie speichert die Zuordnung der vordefinierten Alarmereignisse 43 bis 43^{(m)} zu den vordefinierten Alarmprioritäten 42 bis 42^{(p)} und den vordefinierten Alarmsignalzeitverläufen 45 bis 45⁽ⁿ⁾ sowie die vordefinierten Alarmsignalzeitverläufe 45 bis 45⁽ⁿ⁾ ab. Bei der Speichervorrichtung kann es sich um RAM-Baugruppen (RAM: Random Access Memory) handeln oder aber auch um ROM-Baugruppen (ROM: Read Only Memory). Andere mögliche Realisierungsformen des Speichers wurden bereits weiter oben aufgeführt. Die Speichervorrichtung ist dazu ausgebildet, ein oder mehrere vordefinierte Alarmsignalzeitverläufe und eine oder mehrere Alarmprioritäten so zu speichern, dass jeweils ein Alarmsignalzeitverlauf und jeweils eine Alarmpriorität einem oder mehreren vordefinierten Alarmereignissen zugeordnet sind. Die Zuordnung kann beispielsweise über eine im Speicher abgelegte Tabelle realisiert werden, in der in jeder Zeile ein Alarmereignis sowie die zugeordnete Alarmpriorität sowie ein Zeiger auf einen Speicherbereich eingetragen ist, der den dem Alarmereignis zugeordneten Alarmsignalzeitverlauf enthält.

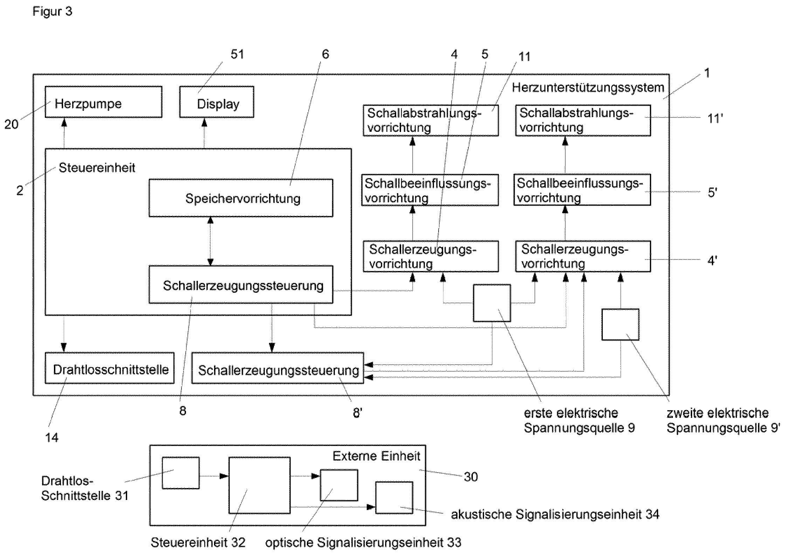

In Figur 3 werden die grundlegenden Komponenten des Herzunterstützungssystems 1 im Systemüberblick dargestellt. Zentrales Element ist die Steuereinheit 2, deren Aufgabe darin besteht, Ereignisse und insbesondere Alarmereignisse 43 zu erkennen und zu verarbeiten sowie Daten zu verarbeiten und Steuerungsaufgaben im Herzunterstützungssystem 1 auszuführen und zu koordinieren. Dazu zählen unter anderem die Steuerung der Herzpumpe 20, die Steuerung der Drahtlosschnittstelle 14 sowie die Steuerung der Schallerzeugungssteuerung 8.

Die erste Schallerzeugungssteuerung 8 hat die Aufgabe, die Schallerzeugung zu steuern. Das Herzunterstützungssystem 1 weist aus Sicherheitsgründen eine weitere, zweite Schallerzeugungssteuerung 8' auf. Sie überwacht die erste Schallerzeugungssteuerung 8 und gibt bei einem festgestellten Funktionsausfall der ersten Schallerzeugungssteuerung 8 mit Hilfe einer zweite Schallerzeugungsvorrichtung 4', einer zweiten Schallbeeinflussungsvorrichtung 5', sowie einer zweiten Schallabstrahlungsvorrichtung 11' ein Warnsignal aus. Eine Möglichkeit, den Funktionsausfall der ersten Schallerzeugungssteuerung 8 zu detektieren besteht darin, dass die erste Schallerzeugungssteuerung 8 im Normalbetrieb an einem elektrischen Ausgang ein mit einem vordefinierten Muster gepulstes Spannungssignal erzeugt, dass von der zweiten Schallerzeugungssteuerung 8' an einem elektrischen Eingang ausgewertet wird. Diese Auswertung kann beispielsweise von einem einfachen Mikrocontroller oder aber auch einer elektronischen Schaltung ausgeführt werden. Die zweite Schallerzeugungssteuerung 8' kann so ausgebildet werden, dass sie eine Abweichung vom vordefinierten Muster, beispielsweise das Ausbleiben eines Pulses, erkennt und daraufhin ein Steuersignal ausgibt, dass die elektrische Ansteuerung eines Lautsprechers freischaltet. Die vorgenannte elektrische Ansteuerung eines Lautsprechers kann durch eine elektronische Oszillatorschaltung realisiert werden, mit der ein gepulstes elektrisches Spannungssignal erzeugt wird, das, sobald es an einen Lautsprecher durchgeleitet wird, zur Erzeugung eines dazu korrespondierenden Schallsignals führt.

Darüber hinaus weist das Herzunterstützungssystem 1 optional ein Display 51, optional eine Drahtlosschnittstelle 14, sowie eine erste elektrische Spannungsquelle 9 und optional eine zweite elektrische Spannungsquelle 9' auf. Die zweite elektrische Spannungsquelle 9' ist so ausgebildet, dass sie die zweite Schallerzeugungsvorrrichtung 4' und die zweite Schallerzeugungssteuerung 8' bei Ausfall der ersten elektrischen Spannungsquelle 9 mit elektrischer Spannung versorgen kann. Bei der Drahtlosschnittstelle 14 kann es sich beispielsweise um eine WLAN-Schnittstelle handeln. Weitere Realisierungsformen für eine Drahtlosschnittstelle 14 wurden bereits weiter oben aufgeführt.

Die externe Einheit 30 ist dazu ausgebildet, über die Drahtlosschnittstelle 31 mit der Drahtlosschnittstelle 14 des Herzunterstützungssystems 1 Daten auszutauschen. Die externe Einheit 30 umfasst darüber hinaus eine eigene Steuereinheit 32 eine optische Signalisierungseinheit 33 und eine akustische Signalisierungseinheit 34. Bei der Drahtlosschnittstelle 31 kann es sich beispielsweise um eine WLAN-Schnittstelle handeln.

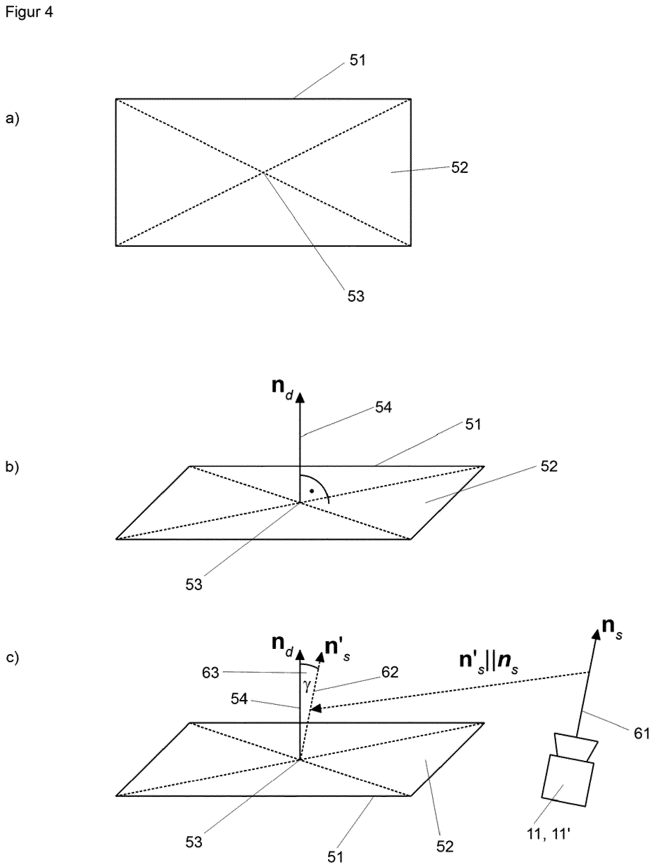

Figur 4 zeigt schematisch das Display 51 des Herzunterstützungssystems 1. Im Flächenschwerpunkt 53 der Displayfläche 52 wird ein Normalenvektor n_{d} 54 definiert, der senkrecht auf der Displayfläche 52 steht und vom Gerät weg weist. Die Schallabstrahlung durch die Schallabstrahlungsvorrichtung 11, 11' erfolgt vorzugsweise so, dass der Winkel γ 63 zwischen dem Normalenvektor n_{d} 54 und dem Vektor nₛ' 62, der parallel zu Hauptschallabstrahlungsrichtung nₛ 61 verläuft, kleiner oder gleich 90° ist.

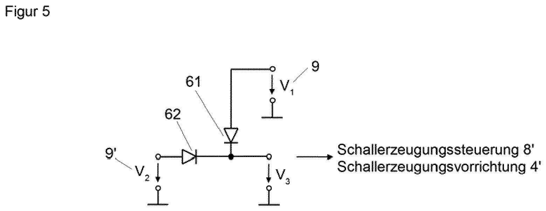

Figur 5 zeigt schematisch eine Möglichkeit wie zwei elektrische Spannungsquellen 9 und 9' miteinander verbunden werden können. Die erste elektrische Spannungsquelle 9 stellt eine elektrische Spannung mit dem Wert V1 zur Verfügung. Die zweite elektrische Spannungsquelle 9', die als Backup-Spannungsquelle dient, stellt eine elektrische Spannung mit dem Wert V2 zur Verfügung. Dabei gilt V1 > V2, beispielsweise V1 = 12V und V2=3V, wobei beide Spannungen V1 und V2 gegen Masse gemessen werden. Die Ausgangsspannung V3 dieser Schaltung dient als elektrische Spannungsversorgung für die Schallerzeugungssteuerung 8', die die Schallerzeugungsvorrichtung 4' ansteuert. Im Normalbetrieb, d.h. bei Anliegen der Spannungswerte V1=12V und V2=3V, ist die Diode 61 geöffnet und die Diode 62 in gesperrt, so dass Strom aus der elektrischen Spannungsquelle 9 in die Schallerzeugungssteuerung 8' und die Schallerzeugungsvorrichtung 4' fließen kann, jedoch nur ein sehr geringer Sperrstrom durch die Diode 62 fließt. Bricht die elektrische Spannungsquelle 9 jedoch zusammen, d.h. V1=0V, sperrt Diode 61 und Diode 62 wird geöffnet. Damit fließt Strom in diesem Fall aus der elektrischen Spannungsquelle 9' in die Schallerzeugungssteuerung 8' und die Schallerzeugungsvorrichtung 4'.

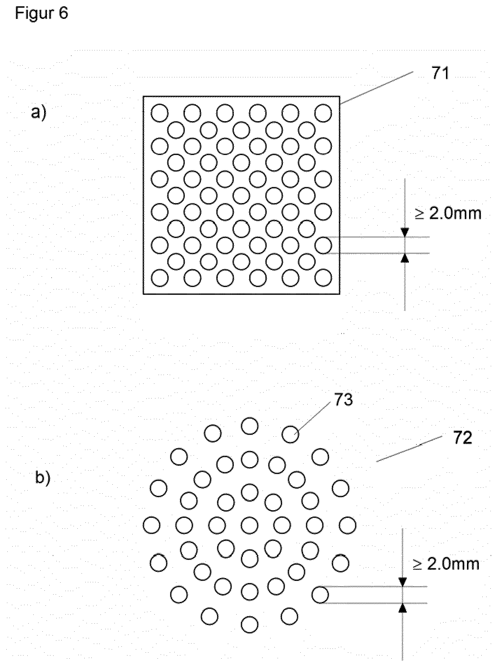

Figur 6 zeigt mögliche Realisierungsformen für die Schallabstrahlungsvorrichtung 11, 11' mit einem rechteckigen Lochfeld 71 und einem kreisförmigen Lochfeld 72 mit den Öffnungen 73, die beispielsweise durch Bohrungen in das Gehäuse des Herzunterstützungssystems 1 realisiert werden. Die Öffnungsdurchmesser der Öffnungen 73 im Lochfeld 71 oder 72 haben vorzugsweise Werte oberhalb von 2mm.

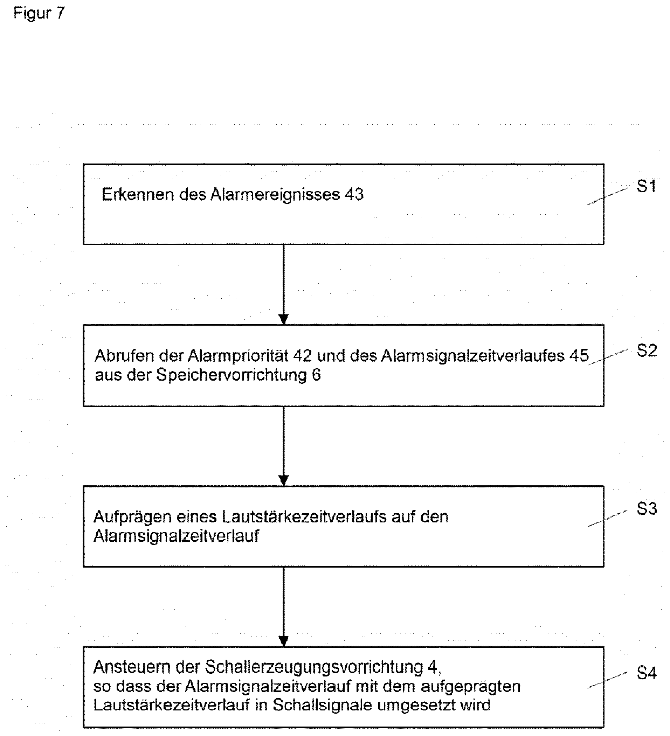

Figur 7 zeigt die Verfahrensschritte zum Abrufen und zur Modifikation der Alarmsignalzeitverläufe. Das beanspruchte Verfahren zu Alarmierung besitzt als ersten Schritt S1 das Erkennen eines Alarmereignisses im Herzunterstützungssystem. Die Erkennung kann sowohl durch eine Software als auch durch eine Hardware realisiert werden. Dabei erfolgt beispielsweise ein Vergleich eines an den Systemeingängen anliegenden Spannungs- oder Stromwertes mit einem Referenzwert oder alternativ ein Vergleich eines berechneten Wertes mit einem Referenzwert. Die Art des Alarmereignisses 43 wird anhand der Art des durchgeführten Vergleichs festgelegt.

In einem zweiten Verfahrensschritt S2 wird aus einer Speichervorrichtung 6 die dem Alarmereignis 43 zugeordnete Alarmpriorität 42 und die der zugeordnete Alarmsignalzeitverlauf 45 abgerufen.

In einem dritten Verfahrensschritt S3 wird dem abgerufenen Alarmsignalzeitverlauf 45, in Abhängigkeit von der Alarmpriorität 42 und von der Zeitdauer seit Eintreffen des Alarmereignisses 43, ein vordefinierter Lautstärkezeitverlauf aufgeprägt. Diese Modifikation erfolgt beispielsweise so, dass die Lautstärke des Alarmsignalzeitverlaufs 45 in einem vordefinierten, von der Alarmpriorität 42 abhängigen, An-Aus-Muster verändert wird. An-Aus-Muster bedeutet, dass die Lautstärke des Signals auf vordefinierte Art und Weise mehrfach vom im Speicher abgelegten Wert auf Null wechselt und umgekehrt. Die Zeitabschnitte, in denen der Lautstärkewert bei Null liegt, werden jeweils als Aus-Phase bezeichnet, die anderen Zeitabschnitte jeweils als An-Phase. Außerdem wird die Lautstärke des Alarmsignals so modifiziert, dass die Lautstärke während der An-Phasen mit zunehmender Zeit gemessen ab Erkennen des Alarmereignisses bis hin zu einem Maximalwert ansteigt.

In einem vierten Verfahrensschritt S4 erfolgt die Ansteuerung der Schallerzeugungsvorrichtung 4 so, dass der Alarmsignalzeitverlauf mit dem aufgeprägten Lautstärkezeitverlauf in ein Schallsignal umgesetzt wird.

## Patentansprüche

1. Herzunterstützungssystem (1) mit einer ersten Steuereinheit (2) und mit mindestens einer ersten Schallerzeugungsvorrichtung (4), wobei die Steuereinheit (2) dazu ausgebildet ist, Ereignisse, und insbesondere Alarmereignisse (43) zu erkennen und zu verarbeiten, sowie Daten zu verarbeiten und Steuerungsaufgaben im Herzunterstützungssystem (1) auszuführen, **gekennzeichnet durch**
- mindestens eine Speichervorrichtung (6), die dazu ausgebildet ist, ein oder mehrere vordefinierte Alarmsignalzeitverläufe (45) und eine oder mehrere Alarmprioriten (42) so zu speichern, dass jeweils ein Alarmsignalzeitverlauf (45) und jeweils eine Alarmpriorität (42) einem oder mehreren vordefinierten Alarmereignissen (43) zugeordnet sind,
- mindestens eine Schallerzeugungssteuervorrichtung (8), die dazu ausgebildet ist, bei Erkennung eines Alarmereignisses (43) dem dem Alarmereignis (43) in der Speichervorrichtung (6) zugeordneten Alarmsignalzeitverlauf (45) jeweils einen vordefinierten Lautstärkezeitverlauf aufzuprägen, der von der in der Speichervorrichtung (6) dem jeweiligen Alarmereignis (43) zugeordneten Alarmpriorität (42) und der Zeitdauer ab Erkennung des Alarmereignisses (43) abhängig ist,
- die vorgenannte Schallerzeugungssteuervorrichtung (8), die dazu ausgebildet ist, bei Erkennung eines Alarmereignisses (43) eine oder mehrere Schallerzeugungsvorrichtungen (4) so anzusteuern, dass sie den vorgenannten Alarmsignalzeitverlauf (45) mit dem vorgenannten aufgeprägten Lautstärkezeitverlauf in Schallsignale umsetzen.

2. Herzunterstützungssystem (1) nach Anspruch 1, wobei das Herzunterstützungssystem (1) eine Herzpumpe (20) umfasst und wobei die Steuereinheit (2) die Herzpumpe (20) steuert.

3. Herzunterstützungssystem (1) nach Anspruch 1 oder 2, **gekennzeichnet durch** mindestens eine zweite, von der ersten getrennt ansteuerbare, elektrisch betriebene Schallerzeugungsvorrichtung (4').

4. Herzunterstützungssystem (1) nach Anspruch 3, **dadurch gekennzeichnet, dass** die Schallerzeugungsvorrichtungen (4, 4') einen Breitbandlautsprecher und einen schmalbandigen Lautsprecher enthalten.

5. Herzunterstützungssystem (1) nach Anspruch 3, **dadurch gekennzeichnet, dass** die Schallerzeugungsvorrichtungen (4, 4') zwei Breitbandlautsprecher enthalten.

6. Herzunterstützungssystem (1) nach einem der vorhergehenden Ansprüche, **gekennzeichnet durch** mindestens eine zweite Steuereinheit (8'), die dazu ausgebildet ist, einen Funktionsausfall der ersten Schallerzeugungssteuereinheit (8) zu detektieren und mindestens eine Schallerzeugungsvorrichtung (4, 4') so anzusteuern, dass die Schallerzeugungsvorrichtung (4, 4') vordefinierte Schallsignale erzeugt.

7. Herzunterstützungssystem (1) nach einem der vorhergehenden Ansprüche, wobei das Herzunterstützungssystem (1) eine erste elektrische Spannungsquelle (9) enthält, **gekennzeichnet durch** eine zweite elektrische Spannungsquelle (9'), die dazu ausgebildet ist, bei Ausfall der ersten elektrischen Spannungsquelle (9) mindestens eine Schallerzeugungsvorrichtung (4') und die zweite Steuereinheit (8') mit Spannung zu versorgen.

8. Herzunterstützungssystem (1) nach einem der vorhergehenden Ansprüche, **gekennzeichnet durch** mindestens eine Schallabstrahlungsvorrichtung (11), die dazu ausgebildet ist, die Schallabstrahlung zu beeinflussen.

9. Herzunterstützungssystem (1) nach Anspruch 8, wobei das Herzunterstützungssystem ein Display (51) enthält, **dadurch gekennzeichnet, dass** der Winkel γ (63) zwischen der Hauptrichtung der Schallabstrahlung (61) durch die Schallabstrahlungsvorrichtungen (11, 11') und dem vom Display (51) wegzeigenden Normalenvektor (63) des Displays (51) einen Wert kleiner oder gleich 90° aufweist.

10. Herzunterstützungssystem (1) nach einem der vorhergehenden Ansprüche, **gekennzeichnet durch** mindestens eine Schallbeeinflussungs-vorrichtung (5, 5'), die dazu ausgebildet ist, die Gewichtung der Schallfrequenzen zu beeinflussen.

11. Herzunterstützungssystem (1) nach einem der vorhergehenden Ansprüche, **gekennzeichnet durch** eine Drahtlosschnittstelle (14), die dazu ausgebildet ist, mit der Drahtlosschnittstelle (31) einer externen Einheit (30) Daten auszutauschen.

12. Herzunterstützungssystem (1) nach einem der vorhergehenden Ansprüche, **dadurch gekennzeichnet, dass** die externe Einheit (30) eine optische Signalisierungseinheit (33) aufweist.

13. Herzunterstützungssystem (1) nach einem der vorhergehenden Ansprüche, **dadurch gekennzeichnet, dass** die externe Einheit (30) eine akustische Signalisierungseinheit (34) aufweist.

14. Verfahren zur Art und Weise der Alarmierung eines Herzunterstützungssystems, **gekennzeichnet durch**
- Erkennen S1 eines Alarmereignisses 43,
- Abrufen S2 der dem Alarmereignis 43 zugeordneten Alarmpriorität 42 und des dem Alarmereignis 43 zugeordneten Alarmsignalzeitverlaufs 45 aus der Speichervorrichtung 6,
- Aufprägen S3 eines Lautstärkezeitverlaufs auf den Alarmsignalzeitverlauf 45, der von der Alarmpriorität 42 und von der Zeitdauer seit Erkennen des Alarmereignisses 43 abhängt,
- Ansteuern S4 der Schallerzeugungsvorrichtung 4, so dass der Alarmsignalzeitverlauf 45 mit dem aufgeprägten Lautstärkezeitverlauf in ein Schallsignal umgesetzt wird.
